# EUROPEAN PATENT APPLICATION

(11) **EP 1 830 183 A1**
(43) Date of publication of application: **05.09.2007**
(21) Application number: 05819502.5
(22) Date of filing: 21.12.2005
(51) Int. Cl.: G01N 27/416, C12M 1/34, C12N 15/09, C12Q 1/68, G01N 27/327, G01N 33/53, G01N 33/543

(54) **PROBE UNIT, APPARATUS FOR IDENTIFYING NUCLEOTIDE REGION AND METHOD OF IDENTIFYING NUCLEOTIDE REGION**

(30) Priority: 21.12.2004 JP 2004369133
(71) Applicant: Kyoto University, Kyoto (JP)
(72) Inventor: OKAMOTO, Akimitsu Kyoto University, Kyoto 6158510 (JP); KAMEI, Taku, Kyoto 6158510 (JP); SAITO, Isao, Kyoto 6078242 (JP)
(74) Representative: Müller-Boré & Partner Patentanwälte
(86) International application number: PCT/JP2005/023539
(87) International publication number: WO 2006/075497

(57) **Abstract**

A probe unit for identifying a target nucleotide region in a target nucleic acid, the unit being provided with an electrode, a probe bound to the electrode and recognizes the target nucleic acid, and a hole-transfer-inducing agent bound to the probe, wherein a nucleotide region corresponding to the target nucleotide region is located between the hole-transfer-inducing-agent-binding site and the electrode-binding end of the probe. A state of the target nucleotide region in the target nucleic acid can be identified by comparing electrochemical signal levels by energy-induced hole transfers before and after the hybridization of such a probe unit with the target nucleic acid.

## Description

### TECHNICAL FIELD

The present invention relates to a probe unit for identifying the state of a nucleotide region at a given site on a target nucleic acid, an apparatus for identifying a nucleotide equipped with said probe, and a method for identifying the nucleotide.

### BACKGROUND ART

A known method for nucleic-acid-base determination using a nucleic acid probe is to hybridize the probe and a target nucleic acid, thereby taking a melting temperature of the hybridization product. More specifically, this method identifies a base taking advantage of slightly different melting temperatures between when a base at a given site on the probe pairs with a corresponding base on the target nucleic acid and when a base at a given site on the probe does not pair with a corresponding base on the target nucleic acid.

In this case, the probe and nucleic acid are typically labeled with a fluorescent substance or radioactive substance. Radioactive labeling requires complicated procedures in a controlled area. Fluorescent labeling requires a special detector, thereby becoming costly.

Further, this method must employ hybridization conditions for each target nucleic acid so as to cause significant melting temperature differences between when corresponding bases form a base pair and when they do not. Furthermore, this method is likely to suffer from detection errors due to non-specific adsorption and instability of base pair formation between bases.

In place of the nucleotide identification method using fluorescent or radioactive labeling, an electrochemical method has started being used.

Patent documents 1 and 2 disclose a method, for example, wherein a nucleic acid probe immobilized on the electrode surface is hybridized with a target nucleic acid, an electrically responsive reagent such as an intercalator of double-stranded nucleic acid is then added to this reaction mixture, and a redox potential derived from the electrically responsive reagent is detected, thereby identifying the presence or absence of the hybridization, and thus a nucleotide at a given site.

However, this method fails to detect mismatches due to almost equal thermodynamic background between when there are a few mismatched bases and when all bases are fully matched, thereby resulting in low detection sensitivity. This method further needs to determine the kind and amount of an electrically responsive reagent for each target nucleic acid, hence causing detection errors. Furthermore, since a target nucleotide is identified based on the presence or absence of hybridization products, this method requires cumbersome procedures to determine optimal hybridization conditions for each target nucleic acid, and is likely to cause detection errors due to variations in hybridization efficiency.

This method employs a technique based on distance-dependant first-stage electron transfer, and can hence accurately identify only comparatively short target nucleic acids. Namely, it cannot be practically applied to nucleic acids derived from a body. The method further requires a comparatively high density of target nucleic acids, thereby becoming expensive.

Patent document 3 discloses a method in which a nucleic acid probe is connected to an electrode via a spacer, a target nucleic acid and the nucleic acid probe are hybridized, an electrically responsive reagent such as an intercalator is added thereto, and a redox potential derived from the electrically responsive reagent is detected, thereby identifying the presence or absence of hybridization products, and thus a nucleotide at a given site. The method disclosed in Patent document 2 enables easy movement of the nucleic acid probe in the reaction mixture due to the use of a spacer, resulting in a high hybridization efficiency with the target nucleic acid for an accordingly enhanced identification efficiency of a specific nucleic acid.

The method disclosed in Patent document 3 is substantially the same as that disclosed in Patent documents 1 and 2, in that it requires the addition of an electrically responsive reagent, and is influenced by hybridization efficiency.

Patent document 4 discloses a method for identifying a specific nucleotide in a target nucleic acid, using probes having reverse sequences complementary to each other in a molecule and a sequence complementary to a target nucleic acid sandwiched between those sequences, and such probes being bound to a redox unit at one end and connected to an electrode at the other end. The probe by itself is capable of having a hairpin structure resulting from hybridization in the molecular, thereby keeping the redox unit and the electrode adjacent to each other. However, when the probes are hybridized with the target nucleic acid, the hairpin structure opens, causing the redox unit to move away from the electrode. The presence or absence of hybridization products is detected based on this change, in the form of an electrochemical signal.

The method disclosed in Patent document 4 does not require the use of an electrically responsive reagent; however optimal hybridization conditions must be employed since the method depends on the hybridization efficiency between the target nucleic acid and the nucleic acid probes.
Patent Document 1: Patent Number 2573443 specification
Patent Document 2: Unexamined Japanese Patent Publication No. 2002-510791
Patent Document 3: Unexamined Japanese Patent Publication No. 2004-61237
Patent Document 4: International Patent Publication No. WO2004-035829

### DISCLOSURE OF THE INVENTION

### PROBLEMS TO BE SOLVED BY THE INVENTION

Objects of the present invention are to provide a highly sensitive probe unit to identify the state of a target nucleotide region in a target nucleic acid without depending on hybridization efficiency, an apparatus for identifying a nucleotide, and a method for identifying the nucleotide region.

### MEANS TO SOLVE THE PROBLEMS

The present inventors conducted extensive research to solve the problems mentioned above, and found the following findings (i) and (ii).
(i) When a target nucleic acid is hybridized with an oligonucleotide probe bound to a hole-transfer-inducing agent at one end and to an electrode at the other end, to which energy such as light is supplied, hole transfer (hole-hopping) occurs via nucleotides having guanine and/or cytosine in the probe, whereby the hole transfer is detected as an electrical signal at the electrode.
   Further, an electrical signal can also be detected as a result of hole transfer by hybridizing a target nucleic acid with a hole-transfer-inducing agent bound at its binding end to a probe with the probe bound to an electrode at the opposite end to the hole transfer-inducing agent binding site, followed by a supply of energy.
   Furthermore, whichever a hole-transfer-inducing agent is bound to, a probe or a target nucleic acid, an electrical signal can also be detected as a result of hole transfer when a hole-transfer-inducing agent is bound after the hybridization, followed by a supply of energy.
(ii) When a nucleotide corresponding to guanine in a target nucleic acid is deoxycitidylic acid or citidylic acid, or a nucleotide corresponding to cytosine in a target nucleic acid is deoxyguanilic acid or guanilic acid, that is, when a G-C base pair is formed, hole transfer readily occurs, whereby a strong electrical signal is detected. However, when a nucleotide corresponding to guanine is other than deoxycitidylic acid or citidylic acid, or a nucleotide corresponding to cytosine is other than deoxyguanilic acid or guanilic acid, that is, when a mismatched base pair is present, hole transfer is less likely to occur, thereby causing a weak electrical signal.
   Utilizing this phenomenon, whether a base in a nucleotide at a given site in a target nucleic acid is cytosine or guanine can be identified with a high sensitivity without relying on hybridization efficiency.
   Further, when a target nucleic acid is not completely complementary to a probe due to deletions and/or insertions, hole transfer is less likely to occur, whereby an observed electrical signal is weak.
(iii) When an electrical signal caused by hole transfer is detected in the same manner as in (i), using as a probe an oligonucleotide derivative containing a nucleotide derivative represented by formula (1) below: 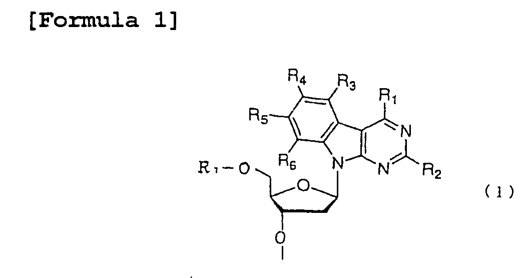
wherein R₁, R₂, R₃, R₄ , R₅ and R₆ each independently represent hydrogen, an amino group, a lower mono-alkylamino group, a lower di-alkylamino group, a hydroxyl group, a lower alkoxy group, a halogen atom, a cyano group, a mercapto group, a lower alkylthio group or an aryl group, hole transfer readily occurs resulting in a remarkable electrical signal in the case of a nucleotide corresponding to the above nucleotide derivative is deoxythymidylic acid; however, when such a nucleotide is other than said acid, hole transfer seldom occurs and hence only causes a small electrical signal.

Utilizing these phenomena, whether or not a given nucleotide in a target nucleic acid is deoxythymidylic acid can be detected with a high sensitivity without relying on hybridization efficiency.

Further, whether or not a nucleotide at a give site in a target nucleic acid is deoxyadenylic acid can be identified by detecting whether or not the corresponding nucleotide in the complementary strand of the target nucleic acid is deoxythymidylic acid.

Furthermore, when a target nucleic acid is not completely complementary to a probe due to deletions and/or insertions, hole transfer is less likely to occur, whereby an observed electrical signal is weak.

The present invention has been accomplished based on the above findings, and provides the following probe unit, an apparatus for identifying a nucleotide region, and a method for identifying a nucleotide region.

Article 1. A probe unit for identifying a target nucleotide region in a target nucleic acid, the probe unit being provided with an electrode, a probe bound to the electrode and recognizes the target nucleic acid, and a hole transfer-inducing agent bound to the probe, wherein a nucleotide region corresponding to the target nucleotide region is located between the hole-transfer-inducing-agent-binding site and the electrode-binding end of the probe.

Article 2. A probe unit for identifying a target nucleotide in a target nucleic acid, the probe unit being of article 1, wherein a nucleotide corresponding to the target nucleotide is located between the hole-transfer-inducing-agent-binding site and the electrode-binding end of the probe.

Article 3. A probe unit of article 1, wherein the probe recognizes the target nucleic acid by the hybridization therewith.

Article 4. A probe unit of article 1, wherein the probe consists of 6 to 30 nucleotides or derivatives thereof.

Article 5. A probe unit of article 1, wherein the hole-transfer-inducing agent is a photosensitizer.

Article 6. A probe unit of article 5, wherein the photosensitizer is capable of causing a photoexcited hole transfer.

Article 7. A probe unit of article 5, wherein the photosensitizer is at least one selected from the group consisting of quinone photosensitizers, flavin photosensitizers, and benzophenone photosensitizers.

Article 8. A probe unit of article 1, wherein the probe is bound to the electrode via a spacer.

Article 9. A probe unit of article 8, wherein the spacer is a substance selected from the group consisting of organic low molecular weight compounds, nucleic acids, and polypeptides.

Article 10. A probe unit of article 8, wherein the spacer has a length of 1 to 3 nm.

Article 11. A probe unit of article 1, wherein the electrode is provided with a plurality of electrode spacers on its spacer-binding face.

Article 12. A probe unit of article 2, wherein the probe is DNA, and a base of the nucleotide corresponding to the target nucleotide in the probe is guanine or cytosine.

Article 13. A probe unit of article 2, wherein the probe is an oligonucleotide derivative including a nucleotide derivative represented by formula (1) below at a site corresponding to the target nucleotide of the target nucleic acid 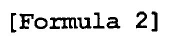 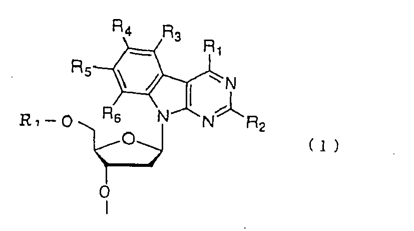 wherein R₁, R₂, R₃, R₄, R₅ and R₆ each independently represent hydrogen, an amino group, a lower mono-alkylamino group, a lower di-alkylamino group, a hydroxyl group, a lower alkoxy group, a halogen atom, a cyano group, a mercapto group, a lower alkylthio group or an aryl group.

Article 14. A probe unit of article 1, wherein a number of continuous nucleotides selected from the group consisting of A and T in the probe is 3 or less.

Article 15. A probe unit of article 1, wherein the nucleotides at the opposite side to the electrode-binding end from the nucleotide to which the hole-transfer-inducing agent is bound in the probe are nucleotides other than G and C.

Article 16. A probe unit of article 1, wherein the hole-transfer-inducing agent is bound to a nucleotide in the probe.

Article 17. An apparatus for identifying a nucleotide region being provided with a substrate, at least one probe unit of any of Articles 1 to 16 and a means for detecting an electrochemical signal from the electrode of the probe, wherein the electrode of the probe unit is positioned on the substrate so as to detect the electrochemical signal.

Article 18. A method for identifying a target nucleotide region, the method comprising:
a first step in which a target nucleic acid and a probe bound to an electrode are hybridized;
a second step in which a hole-transfer-inducing agent is directly or indirectly bound to the probe or target nucleic acid before or after the first step, during which the hole-transfer-inducing agent is bound to the probe so that a nucleotide region corresponding to the target nucleotide region is located between the hole-transfer-inducing-agent-binding site and electrode-binding site of the probe, or the hole-transfer-inducing agent is bound to the target nucleic acid so that the target nucleotide region is located between a site corresponding to the electrode-binding site of the probe and the hole-transfer-inducing-agent-binding site in the target nucleic acid; and
a third step in which a hole transfer from the hole-transfer-inducing-agent-binding site to the electrode in the probe is caused by an energy supply to the above hybridized product, an electrochemical signal detected from the electrode is detected, the signal is compared with an electrochemical signal detected from the electrode caused by an energy supply to the probe before the hybridization, and whether or not the target nucleotide region in the target nucleic acid is completely complementary to the corresponding region in the probe is identified based on this comparison.

Article 19. A method of article 18, wherein the hole-transfer-inducing agent is bound to the probe.

Article 20. A method of article 18, wherein the second step is performed before the first step.

Article 21. A method of article 18, wherein the target nucleotide region consists of a single target nucleotide.

Article 22. A method of article 18, wherein the probe consists of 6 to 30 nucleotides or derivatives thereof.

Article 23. A method of article 18, wherein the hole-transfer-inducing agent is a photosensitizer.

Article 24. A method of article 23, wherein the photosensitizer is capable of causing a photoexcited hole transfer.

Article 25. A method of article 23, wherein the photosensitizer is at least one selected from the group consisting of quinone photosensitizers, flavin photosensitizers, and benzophenone photosensitizers.

Article 26. A method of article 18, wherein the probe is bound to the electrode via a spacer.

Article 27. A method of article 26, wherein the spacer is a substance selected from the group consisting of organic low molecular weight compounds, nucleic acids, and polypeptides.

Article 28. A method of article 26, wherein the spacer has a length of 1 to 3 nm.

Article 29. A method of article 18, wherein the electrode is provided with a plurality of electrode spacers on its spacer-binding face.

Article 30. A method of article 21, wherein the probe is DNA, and a base of the nucleotide corresponding to the target nucleotide in the probe is guanine or cytosine, the method identifying whether the base of the target nucleotide in the target nucleic acid is cytosine or guanine in the third step.

Article 31. A method of article 21, wherein the probe is an oligonucleotide derivative containing a nucleotide derivative represented by formula (1) below at a site corresponding to the target nucleotide in the target nucleic acid, the method identifying whether or not the base of the target nucleotide in the target nucleic acid is thymine in the third step 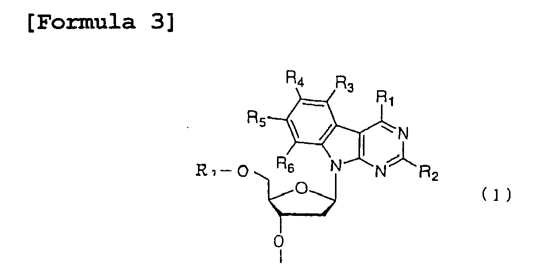 wherein R₁, R₂, R₃, R₄, R₅ and R₆ each independently represent hydrogen, an amino group, a lower mono-alkylamino group, a lower di-alkylamino group, a hydroxyl group, a lower alkoxy group, a halogen, a cyano group, a mercapto group, a lower alkylthio group or an aryl group.

Article 32. A method of article 18, wherein a number of continuous nucleotides selected from the group consisting of A and T in the probe is 3 or less.

Article 33. A method of article 18, wherein the nucleotides at the opposite side to the electrode-binding end from the nucleotide to which the hole-transfer-inducing agent is bound in the probe are nucleotides other than G and C.

Article 34. A method of article 18, wherein the hole transfer-inducing agent is bound to the nucleotide in the probe.

Article 35. A method of article 18, wherein energy is irradiated at a power density of 1 to 100 mW · cm⁻².

Article 36. A method of article 23, wherein light having a wavelength of 300 to 600 nm is irradiated.

Article 37. A method for identifying a target nucleotide region comprising:
a step of hybridizing the probe in the probe unit of article 12 and a target nucleic acid; and
a step of detecting an electrochemical signal from the electrode by an energy supply to the probe, comparing the signal with an electrochemical signal detected from the electrode when energy is supplied to a probe with which the target nucleic acid is not hybridized, and identifying whether or not a target nucleotide region in the target nucleic acid is completely complementary to a corresponding region in the probe based on this comparison.

Article 38. A method of article 37, wherein the target nucleotide region consists of a single nucleotide, the method identifying in the identification step whether or not the base of the target nucleotide in the target nucleic acid is cytosine or guanine by identifying whether or not the target nucleotide in the target nucleic acid is complementary to the corresponding region in the probe.

Article 39. A method of article 37, wherein energy is irradiated at 1 to 100 mW. cm⁻².

Article 40. A method of article 37, wherein the energy is light, and light having a wavelength of 300 to 600 nm is irradiated.

Article 41. A method for identifying a target nucleotide region comprising:
a step of hybridizing the probe in the probe unit of article 13 and a target nucleic acid; and
a step of detecting an electrochemical signal from the electrode by an energy supply to the probe, comparing the signal with an electrochemical signal detected from the electrode when energy is supplied to a probe with which the target nucleic acid is not hybridized, and identifying whether or not a target nucleotide region in the target nucleic acid is completely complementary to a corresponding region in the probe based on this comparison.

Article 42. A method of article 41, wherein a target nucleotide region consists of a single nucleotide, the method identifying in the identification step whether or not the base of the target nucleotide in the target nucleic acid is thymine by identifying whether or not the target nucleotide in the target nucleic acid is complementary to the corresponding region in the probe.

Article 43. A method of article 41, wherein energy is irradiated at 1 to 100 mW · cm⁻².

Article 44. A method of article 41, wherein the energy is light, and light having a wavelength of 300 to 600 nm is irradiated.

Article 45. A method for producing a probe unit for identifying a target nucleotide region in a target nucleic acid, the method comprising the steps of binding a hole-transfer-inducing agent directly or indirectly to a probe that recognizes a target nucleic acid, and of binding an end of the probe to an electrode, the hole-transfer-inducing agent being bound during the hole-transfer-inducing-agent binding step so that a nucleotide region corresponding to the target nucleotide region is located between the hole-transfer-inducing-agent-binding site and electrode-binding end of the probe.

### EFFECTS OF THE INVENTION

The method for identifying a nucleotide region of the present invention detects an electrical signal caused by energy-stimulated hole transfer in a nucleic acid probe, and identifies a target nucleotide in a target nucleic acid by taking advantage of phenomena in different hole transfer efficiencies depending on corresponding nucleotides, thereby providing extremely sensitive nucleotide identification. Further, when a probe does not hybridize with a target nucleic acid one-to-one due to a deletion or insertion present in a target nucleic acid, hole transfer efficiency is impaired, and hence the present invention can also detect such a deletion and insertion in addition to the identification of a nucleotide region in a target nucleic acid.

More specifically, if there are inconsistencies such as an even single mismatch between a given nucleotide in the probe and corresponding nucleotide during the hybridization between the target nucleic acid and the probe, hole transfer does not or hardly occurs thereafter, thereby detecting an even single base difference. Given this, the method can also be applied for the detection of single nucleotide polymorphisms. Further, when the target nucleic acid is present in an extremely small amount, e.g., in the order of zeptomoles (10⁻²¹ mol), the method can identify the target nucleotide therein. Furthermore, since the method detects a signal caused by hole transfer, it is free from the influence of hybridization efficiencies.

The method of the present invention does not require employing hybridization conditions for an individual target nucleic acid, and is thereby less likely to cause detection errors due to differences in hybridization conditions.

Since the method of the present invention detects an electrical signal caused by hole transfer, it does not need the addition of an electrically responsive reagent. For this reason, detection errors caused by differences in the kind and amount of electrically responsive reagent do not occur. Further, the method does not require the cumbersome steps of adding an electrically responsive reagent or removing an excess of such a reagent.

### BRIEF DESCRIPTION OF THE DRAWINGS

[FIG. 1] A graph showing the results of nucleotide identifications using probe 1.
[FIG. 2] A graph showing the results of nucleotide identifications using probe 2.
[FIG. 3] Formulae showing a method for synthesizing an MDA-containing deoxyribonucleoside derivative.
[FIG. 4] Formulae showing a method for synthesizing an MDA-containing oligonucleotide derivative.
[FIG. 5] A graph showing the results of nucleotide identifications using probe 3.
[FIG. 6] A graph showing the results of nucleotide identifications using probe 4.
[FIG. 7] Figures showing the results of classifying a panel of 25 people into T/T homozygous, C/C homozygous or C/T heterozygous, based on nucleotide identifications using G probe and MDA probe.

### BEST MODE FOR CARRYING OUT THE INVENTION

The present invention is described in detail below.

### (I) Probe Unit

The probe unit of the present invention is to identify a target nucleotide region in a target nucleic acid, and is provided with an electrode, a probe bound to the electrode and recognizes the target nucleic acid, and a hole transfer-inducing agent bound to the probe. On the probe, a nucleotide region corresponding to the target nucleotide region is located between the probe's electrode-binding end and hole-transfer-inducing-agent-binding site.

### Probe

The probe is an oligonucleotide that recognizes a target nucleic acid or derivative thereof, and includes those which recognize the target nucleic acid when hybridized therewith under certain conditions.

The probe encompassed in the probe unit of the present invention is an oligonucleotide or derivative thereof (the first probe), or an oligonucleotide derivative containing a nucloetide derivative represented by formula (1) above (the second probe).

When the first probe is used to detect whether it matches or mismatches with a target nucleic acid, namely, to identify the kind of target nucleotide in a target nucleic acid, the first probe can have nucleotides substantially or fully complementary to the corresponding region in the target nucleic acid, except the nucleotide corresponding to the target nucleotide to be identified in the target nucleic acid. In the first probe, the nucleotide base corresponding to the nucleotide to be identified is guanine or cytosine.

When the second probe is used to detect whether it matches or mismatches with a target nucleic acid, namely, to identify the kind of target nucleotide in a target nucleic acid, the second probe can also have nucleotides substantially or fully complementary to the corresponding region in the target nucleic acid, except the nucleotide corresponding to the target nucleotide to be identified in the target nucleic acid. In the second probe, a nucleotide derivative represented by formula (1) corresponds to the target nucleotide in the target nucleic acid. Among the nucleotide derivatives of formula (1), compounds wherein R₁ or R₂ represent an amino group are preferable, and those wherein R₁ represents an amino group, R₄ represents a methoxy group, R₂, R₃, R₅ and R₆ are each hydrogen (methoxy benzodeazaadenine; MDA containing-nucleotide derivatives) are more preferable. The structure of an MDA-containing nucleotide derivative is shown below.

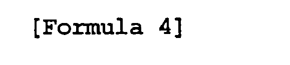

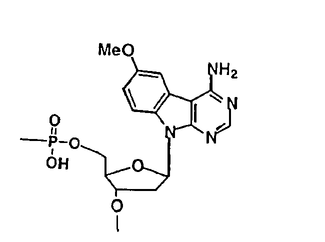

When the first and second probes are to detect excessive numbers of nucleotides in a target nucleic acid against the probes, namely, nucleotide insertions, nucleotides in the target nucleic acid bulge out of the double strands when hybridized. In such cases, the probes can be substantially or fully cmplementary to the region, excluding the bulge region, of the target nucleic acid.

When the first and second probes are to detect nucleotide shortage in a target nucleic acid against the probes, namely, nucleotide deletions, nucleotides in the probes bulge out of the double strands when hybridized. In such cases, the probes can be substantially or fully complementary to the region, excluding the bulge region, of the target nucleic acid. In the first and second probes, when nucleotides located at the opposite side to the electrode-binding end from the nucleotide to which a hole-transfer-inducing agent is bound, more specifically, nucleotides located on the open-end side from the hole-transfer-inducing-agent-binding site are preferably G bases, inosine nucleotides can preferably be employed in place of G bases. Such an employment can facilitate a further efficient hole transfer from the hole-transfer-inducing agent in an electrode direction.

When the hybridization of the first or second probe with a target nucleic acid results in four or more sequences of the base A or T, the nucleotide derivatives of formula (1) (representative of MDA-containing nucleotides) are preferably employed in place of any one of the base A and T. Such a substitution can inhibit decreases in hole transfer efficiency caused by the presence of A or T. In this case, the nucleotide derivatives of formula (1) are irrelevant to nucleotide identification in the target nucleic acid.

The first and second probe may be DNA or RNA, but DNA is preferable for readily induced hole transfer and high detection sensitivity. Modified DNAs such as phosphorothioate DNA, H-phosphonate DNA, and modified RNAs such as 2'-O-RNA, phosphorochioate RNA can further be used. A chimeric nucleotide sequence consisting of DNA and RNA can also be used.

The probe length is not limited, and includes those termed polynucleotide in addition to those typically termed oligonucleotide. The probe length is not limited; however, a probe has preferably about 6 to about 30 bases, and more preferably about 6 to 20 bases. A probe length within the above range which raises the melting temperature (Tm) to room temperature or above, enables the probe to form stable base pairs with a target nucleic acid at room temperature, and causes a sufficiently strong electrochemical signal in response to an energy supply.

In the first and second probes, a region consisting of nucleotides or derivatives thereof corresponding to the nucleotide region to be identified only needs to be located between the hole-transfer-inducing-agent-binding nucleotide and the electrode-binding end. However, such a region located extremely close to the end tends to cause smaller differences in electrochemical signals between when the probes match with the target nucleic acid and when they mismatch, etc. with the target nucleic acid. For this reason, it is desirable that said region be designed to be located as close to the center of the probe as possible.

### Hole-Transfer-Inducing Agent

The hole transfer-inducing agent may be any agent capable of inducing hole transfer in a probe to which the agent is bound by the irradiation of an energy such as light, electro beam, etc. Representative hole-transfer-inducing agents are photosensitizers. The photosensitizer used is not limited, and known products can be used. Examples include quinone molecules such as anthraquinone, flavin molecules such as lumiflavin, benzophenone molecules such as cyanobenzophenone, nucleic acid-binding dyes such as ethidium bromide, nucleic acid-binding metal complexes such as triphenyl ruthenium, etc.

Quinone-, flavin-, and benzophenone sensitizers are preferable among these for their higher detection sensitivities. Further, since quinone molecules and flavin molecules are intercalators, they are stably maintained at its position as an intercalate between double strands after hybridization with a target nucleic acid. Quinone sensitizers are more preferable, with anthraquinone being particularly preferable.

The hole-transfer-inducing agent can be bound to a nucleotide in the probe located at the opposite side to the electrode-binding end, sandwiching the nucleotide region corresponding to the nucleotide region in the probe to be identified. The hole-transfer-inducing agent is preferably bound to an inner nucleotide rather than to the end nucleotide of the probe. The hole transfer-inducing agent, when preferably bound as such, can be positioned between the double strands, and stably maintained in its position.

Since energy-induced hole transfer can occur bidirectionally in the probe from the 5' end side to the 3' end side, and from the 3' end side to the 5' end side, the hole-transfer-inducing agent in the probe may be bound to the 5' end side or to the 3' end side of a site corresponding to the target nucleotide.

The hole-transfer-inducing agent may directly be bound to the probe, but a nucleotide in the probe can be, for example, aminated and the agent can alternatively be bound to this amino group.

The binding amount of the hole-transfer-inducing agent is preferably about 1 to about 20 molecules, and more preferably about 1 to about 3 molecules, per probe. The binding amounts within this range are capable of inducing hole transfer in an oligonucleotide, and enhancing the base identification sensitivity. More specifically, an extremely large amount of the hole-transfer-inducing agent induces hole transfer even when mismatched base pairs and the like are present, thereby failing to specifically detect matched base pairs; however, the amounts within the above range do not cause such a problem.

### Spacer

A probe is preferably bound to an electrode via a spacer. The spacer functions to prevent the probe, which might contact the electrode by bending, etc., from inhibiting hole transfer throughout the entire probe. The spacer molecule is not limited, and usable examples include organic low molecular weight compounds; polypeptides; nucleic acids, etc.

The spacer only needs to have a functional group on its one end for binding to the electrode. Examples of such a functional group include a thiol group, a silicic acid residue, a phosphoric acid residue, an amino group, a biotin residue, etc.

The spacer preferably has a length of 1 to 3 nm. Spacers having a length within this range can function sufficiently as a spacer, and do not affect hole transfer to the electrode from the end of the probe. Said length is equivalent to the length of about C₂ to about C₁₀ when the spacer, excluding the end functional group, is linear saturated hydrocarbon. Said length is equivalent to the length of about 1 to about 5 nucleotides when the spacer is a nucleic acid. Said length is equivalent to the length of about 1 to about 10 amino acids when the spaces is a polypeptide.

The spacer, excluding the end functional group, is preferably a linear saturated hydrocarbon having a length of about C₂ to about C₁₀, and particularly about C₄ to about C₈. Specific examples of such spacers include C₂ to C₁₀, particularly C₄ to C₈, alkyl thiol and alkyl amine.

### Electrode

The electrode is to detect a photocurrent flowing in a probe.

The electrode material is not limited, and examples include noble metals such as gold, platinum, platinum black, palladium, rhodium, etc.; carbons materials such as graphite, glassy carbon, etc.; metal oxides such as titanium oxide, tin oxide, manganese oxide, lead oxide, ITO, etc.; semiconductors such as silicone, germanium, gallium arsenide, zinc oxide, etc.; titanium, etc.

This electrode can be used as a working electrode when, for example, a two-electrode or three-electrode electrochemical cell is used as a means of detecting an electrochemical signal.

The area of the electrode is not limited, and may typically be about 1 mm² to about 1 cm². The areas within this range can bind a probe capable of passing an electrical signal strong enough to be detected on a single electrode.

On a single electrode, the probe can be bound in an amount of typically about 1 fmol to about 1 µmol, and preferably about 1 pmol to about 10 nmol, per 1 mm². The amounts within this range can detect an electrochemical signal strong enough to detect a single base difference, a single base insertion or deletion.

### Electrode Spacer

The probe unit of the present invention is preferably provided with a plurality of electrode spacers on the probe binding face of an electrode. These electrode spacers function to prevent the probe, which directly contacts the electrode, from inhibiting hole transfer throughout the entire probe.

The electrode spacers preferably have a functional group on one end for binding to the electrode, and have a hydrophilic functional group such as a hydroxyl group, an amino group, a carboxyl group, etc. on the other end. The hydrophilic functional group at the opposite end to the electrode-binding end of the electrode spacers enables the prevention of unspecific interactions between the electrode spacers and a probe and/or a target nucleic acid, and does not cause the inhibition of photo-induced hole transfer by the electrode spacers.

The electrode spacers preferably have a length of about 1 to about 3 nm. Electrode spacers having a length within the above range are able to work sufficiently as electrode spacers. Said length is equivalent to the length of about C₂ to C₁₀ when the spacers, excluding the end functional group, are linear saturated hydrocarbons. Alternatively, said length is equivalent to the length of about 1 to about 5 nucleotides when the spacers are nucleic acids. Said length is also equivalent to the length of about 1 to about 10 amino acids when the spacers are polypeptides.

The electrode spacers, excluding the end functional group, are preferably linear saturated hydrocarbons having a length of about C₂ to about C₁₀, and particularly about C₄ to about C₈. Specific examples of such electrode spacers include C₂ to C₁₀, particularly C₄ to C₈, mercapto-alkanol and alkyl amines.

Typically about 1 to about 1000 molecules, and preferably about 100 to about 500 molecules of the electrode spacer, per probe molecule, can be bound to the electrode. Molecule numbers within the above range can provide adequate effects as electrode spacers.

### (II) Process for Producing Probe Unit

The process for producing the probe unit of the present invention comprises the steps of binding a hole-transfer-inducing agent to a target nucleic acid-recognizing probe, and binding one end of the probe to an electrode, the hole-transfer-inducing agent being bound during its binding step so that a nucleotide region corresponding to the target nucleotide region is located between the hole transfer-inducing agent-binding site and the electrode-binding end of the probe.

The probe and the target nucleotide region, and the probe and the electrode, can usually be bound by covalent bonds.

### (III) Apparatus for Identifying Nucleotide

The apparatus for identifying a nucleotide of the present invention is provided with a substrate, one or more of the above probe unit, and a means of detecting an electrochemical signal from an electrode of the probe unit(s). The probe unit(s) is/are disposed on the substrate so as to detect the electrochemical signal from the electrode.

The substrate supports the probe unit, and also functions, in case a plurality of the probe units are used, to insulate between the electrodes of these probe units.

The substrate material is not limited as long as it is an electrically insulating material. Examples include glasses, cements, ceramics such as china and porcelain, etc.; polymers such as polyethyleneterephthalate, cellulose acetate, polycarbonate, polystylene, polymethyl methacrylate, etc.; silicone, activated charcoal, etc.

The electrodes of the probe units are insulated from each other by the substrate material, and are connected to a means of detecting the electrochemical signal.

The means of detecting the electrochemical signal is not limited as long as it is capable of detecting a very small current caused by hole transfer in the form of current, voltage, Coulomb energy, resistance value, etc. Examples of means capable of detecting such small currents include two- or three-electrode electrochemical cells. In such a case, the means for detecting electrochemical signals is provided with a reference electrode or a reference electrode and a counter electrode, as well as a voltmeter or an ammeter, and constitutes an electrochemical cell together with the electrodes of the probe(s) as working electrodes, thereby detecting a photocurrent.

### (IV) Method for Identifying Nucleotide

The method for identifying a target nucleotide region of the present invention comprises:
the first step in which a target nucleic acid and a probe bound to an electrode are hybridized;
the second step in which a hole-transfer-inducing agent is directly or indirectly bound to the probe or target nucleic acid before or after the first step, during which the hole-transfer-inducing agent is bound to the probe so that the nucleotide region corresponding to the target nucleotide region is located between the hole-transfer-inducing-agent-binding site and electrode-binding site of the probe, or the hole-transfer-inducing agent is bound to the target nucleic acid so that the target nucleotide region is located in the target nucleic acid between a site corresponding to the electrode-binding site and the hole-transfer-inducing-agent-binding site of the probe; and
the third step in which a hole transfer from the-hole transfer-inducing-agent-binding site to the electrode in the probe is caused by an energy supply to the above hybridized product, an electrochemical signal detected from the electrode is detected, and the electrochemical signal is compared with an electrochemical signal detected from the electrode by an energy supply to the probe before the hybridization, thereby identifying whether or not the target nucleotide region in the target nucleic acid is completely complementary to the corresponding region in the probe.

The hole transfer-inducing agent may be bound to either the probe or the target nucleic acid. Whichever the agent is bound to, energy supplied is used for hole generation by the hole-transfer-inducing agent, causing hole transfer toward the electrode in the double strands.

When the hole-transfer-inducing agent is bound to the probe, the agent may be bound so that a nucleotide region corresponding to the target nucleotide region is located between the hole-transfer-inducing-agent-binding site and the electrode-binding site of the probe.

When the hole-transfer-inducing agent is bound to the target nucleic acid, the agent may be bound so that a nucleotide region to be identified is located between a site corresponding to the electrode-binding site and the hole-transfer-inducing-agent-binding site of the probe.

The hole-transfer-inducing agent may directly be bound; however, when bound after the hybridization of the probe and the target nucleic acid, a DNA binder such as Hoechst 33258, Mitomycin, Cisplatin, Distamycin, etc. is bound between the double strands, and the hole-transfer-inducing agent is bound to the DNA binder. Further, when the hole-transfer-inducing agent is the DNA binder, the agent only needs to be bound between the double strands.

The probes, spacer, electrode, electrode spacers, and hole-transfer-inducing agent are as described in the probe unit of the present invention.

When the hole-transfer-inducing agent is bound to the probe before the first step, the probe unit of the present invention described above can also be used without performing the second step. In such a situation, the method for identifying a nucleotide region of the present invention can be divided into the following first and second methods.

The first method for identifying a nucleotide of the present invention comprises the steps of hybridizing a target nucleic acid and the first probe (oligonucleotide) of the probe unit of the present invention; and of identifying whether or not a target nucleotide region in the target nucleic acid is completely complementary to the corresponding region of the probe by detecting an electrochemical signal from the electrode caused by an energy supply to the probe, and comparing the detected signal with an electrochemical signal detected from the electrode caused by an energy supply to a probe with which the target nucleic acid is not hybridized. Representatively, the method detects whether or not the target nucleotide in the target nucleic acid is complementary to the corresponding nucleotide of the probe, thereby identifying whether or not the base of the target nucleotide in the target nucleic acid is guanine or cytosine.

When the base of a specific nucleotide in the probe corresponding to the target nucleotide to be identified in the target nucleic acid is guanine, the first method identifies whether or not the base in the target nucleotide in the target nucleic acid is cytosine. When the base of a specific nucleotide in the probe is cytosine, the first method identifies whether or not the base of the target nucleotide in the target nucleic acid is guanine.

The second method for identifying a nucleotide of the present invention employs a probe unit provided with the second probe comprising an oligonucleotide derivative containing a nucleotide derivative represented by formula (1), in place of the first probe used in the first identification method above. Representatively, the second method detects whether or not the target nucleotide in the target nucleic acid is complementary to the corresponding nucleotide in the probe, thereby identifying whether or not the base of the target nucleotide in the target nucleic acid is thymine.

### Target Nucleic Acid

Any single-strand nucleic acid can be a target nucleic acid. The target nucleic acid may be DNA or RNA, but DNA nucleic acids have a higher detection sensitivity. When DNA or a derivative thereof is used as a probe, and DNA is used as a target nucleic acid, a nucleotide can be identified with a very high sensitivity.

In the situation where a probe containing a nucleotide derivative represented by formula (1) is used, the nucleotide derivative, when paired with deoxythymidine, causes a strong hole transfer, whereby a target nucleic acid is identified as DNA.

When a target nucleic acid is hybridized with a probe after being amplified by a known method such as PCR, detection sensitivity is enhanced. For such cases, the nucleotide identification method of the present invention can include a step of amplifying a target nucleic acid before the hybridization step.

Further, a target nucleic acid can also be used in the form of a human blood sample, and samples containing impurities, such as, e.g. nucleic acid extract from food which may contain microorganisms, etc. If such is the case, a double-stranded nucleic acid can be denatured to single-stranded nucleic acids by heating a sample at about 60 to about 100 °C, preferably followed by PCR amplification for use.

### Hybridization Step

To perform hybridization, a target nucleic acid in a concentration of, for example, about 1 nM to about 1 mM may be brought into contact with the probe of the present invention.

Hybridization conditions may vary depending on the complementarity, etc. between an oligonucleotide in the probe and the target nucleic acid, but hybridization can be performed under varying conditions, for example, at a temperature of about 5 to about 40 °C for about 30 seconds to about 1 hour in a neutral buffer solution in a concentration of 10 to 100 mM (representatively, a 50 mM sodium phosphate buffer (pH 7.0)). Further, a conventional nucleotide identification method utilizing hybridization is likely to result in detection errors if end-binding nucleic acids are not washed off after the hybridization; however, according to the method of the present invention, residual end-binding nucleic acids do not inhibit hole transfer inside a nucleic acid. Accordingly, washing after hybridization is not always necessary, but it may be done if desired. The washing may be done under varying conditions, for example, at a temperature of about 5 to about 40 °C for about 30 seconds to about 1 hour in a neutral buffer solution in a concentration of 10 to 100 mM.

Even without using such a hybridization solution, coarse samples such as blood samples, nucleic acid extracts from food can be brought directly into contact with the probe of the present invention. In this case, the coarse sample may be brought into contact with the probe at a temperature of about 5 to about 40 °C for about 30 seconds to about 1 hour.

### Light Irradiation Step

To the probe with which the target nucleic acid is thus hybridized, energy such as light is supplied typically at a power density of about 1 to 100 mW · cm⁻², preferably at a power density of about 5 to about 50 mW cm⁻² for about several msec to about 1 minute. The power densities within said range can induce adequate hole transfer. Power densities higher than those in said range are only expensive.

When the energy used is in the form of light, the wavelength is not limited, and may be selected to be suitable for the photosensitive region of the hole-transfer-inducing agent (in this case, a photosensitizer), with, e.g. about 300 to about 600 nm of UV light being preferable. Too short a wavelength may induce an electrical current from the light absorbed by a polynucleotide and/or a substrate, leading to possible errors; however the wavelengths within said range do not cause such problems.

### Detection and Identification of Electrochemical Signal

In the identification step, an electrochemical signal detected by an energy supply to the probe, with which the target nucleic acid is hybridized, is compared with an electrochemical signal detected by an energy supply, under the same conditions, to the probe with which the target nucleic acid is not hybridized. This step is performed because absolute values of electrochemical signals vary depending on the sequence and length of the target nucleic acid and probes.

In the first method which employs an oligonucleotide probe, the base of the target nucleotide in the target nucleic acid can be identified as cytosine or guanine when a signal value (electrical current, voltage, etc.) after hybridization is, for example, 1.5 or higher, and preferably 3 or higher, taking a signal value before hybridization with the target nucleic acid as being 1. A signal value lower than these values can be indicative of the base of the target nucleotide being something other than cytosine or guanine, or as not being hybridized with the probe one-to-one due to the presence of a deletion or insertion.

In the second method which employs an oligonucleotide derivative probe, the target nucleotide in the target nucleic acid can be identified as deoxythymidilic acid when a signal value (electrical current, voltage, etc.) after hybridization is, for example, 1.5 or higher, and preferably 3 or higher, taking a signal value before hybridization with the target nucleic acid as being 1. A signal value lower than these values can be indicative of the base of the target nucleotide being something other than thymine, or as not being hybridized with the probe one-to-one due to the presence of a deletion or insertion.

The probe with which the target nucleic acid is hybridized can be reused as a single strand by treating with heat or formamide.

### EXAMPLES

The present invention is described in detail below with reference to EXAMPLES, but is not limited to these EXAMPLES.

### < Measurement Method >

¹H NMR was conducted using a Varian Mercury 400 (400 MHz) spectrometer. Coupling constant (J value) is expressed in hertz. Chemical shift is expressed in ppm to the downstream from tetramethylsilane, using residual chloroform as internal standard (δ = 7.24 in H NMR).

FAB mass spectrometry was conducted using a JEOL JMS HX-110A spectrometer. A DNA synthesis reagent was purchased from Glen Research.

The amount of oligodeoxynucleotide was determined using a MALDI-TOF MS (Perseptive Voyager Elite, acceleration voltage 21kV, negative mode), with 2',3',4'-trihydroxyacetophenone as a matrix, and T8 ([M-H⁻]2370.61) and T17 ([M-H⁻]5108.37) as oligonucleotide internal standards.

Reversed phase HPLC was performed using a CHEMOCOBOND 5-ODS-H column (10 x 150 mm, 4.6 x 150 mm) provided with Gilson chromatograph: Model 305, and a 254 nm UV detector: Model 118.

### EXAMPLE 1

### Synthesis of 2-Anthraquinone Carboxylic Acid N-Hydroxy Succineimide Ester

2-Anthraquinone carboxylic acid N-hydroxy succineimide ester, a photosensitizer, was synthesized as follows.

EDCI (152 mg, 0.79 mmol) was added to an acetonitrile (6 ml) solution containing 2-anthraquinone carboxylic acid (200 mg, 0.79 mmol) and N-hydroxy succineimide (91 mg, 0.79 mmol), and the mixture was stirred for 2 hours at room temperature. The reaction mixture was evaporated, and the raw product thereby was purified by silica gel column chromatography (chloroform/methanol = 100:1), thereby obtaining 2-anthraquinone carboxylic acid N-hydroxy succineimide ester in the form of a light yellow solid.

The product was subjected to ¹H NMR (CDCl₃, 400MHz), detecting chemical shifts, δ 9.08 (d, J=1.6 Hz, 1H), 8.52 (dd, J=8.2, 1.8 Hz, 1H), 8.47 (d, J=8.0 Hz, 1H), 8.39-8,34 (2H), 7.88-7.85 (2H), and 2.96 (s, 4H).

FABMS was further performed, obtaining the result of m/e (%) 350 [(M+H)⁺]

Furthermore, HRMS was performed on C₁₉H₁₂NO₆, detecting a calculative [(M+H)⁺]350.0665 at the position 350.0663.

### EXAMPLE 2

### Anthraquinone-Modified Oligodeoxynucleotide

DNAs were synthesized by phosphoramidite method, using an Applied Biosystem 392 DNA/RNA synthesizer. The incorporation of an amino group and disulfide group was performed using Amino-Modifier C2 T2 and Thiol-Modifier C6 S-S (Glen Research), respectively. The synthesized DNAs were subjected to reversed phase HPLC on a 5-ODS-H column (10 x 150 mm), and eluted with 0.1 M triethylamine acetate (pH7.0) for 30 minutes with a linear gradient of 5 to 20 % acetonitrile at a flow rate of 3.0 mL/min.

Synthesized by this method were oligodeoxynucleotides 5'-d(T^{amino}UCACTTCAGTG) - (CH₂) ₆-S-S- (CH₂) ₆-T-3' (nucleic acid part is represented by sequence number 1) and 5' -d(T^{amino}UACACTGAAGTG) - (CH₂)₆-S-S- (CH₂) ₆-T-3' (nucleic acid part is represented by sequence number 2), wherein U was amino-modified and contained a disulfide group at their 3' end.

To a 50 mM sodium phophate buffer (pH 8.0) solution (25 µM, total amount 100 µL), containing these two kinds of oligodeoxynucleotides respectively, was added a 1:1 DMF/dioxane (40 µL) solution (50 mM) of 2-anthraquinone carboxylic acid N-hydroxy succineimide ester obtained in EXAMPLE 1, followed by incubation at room temperature for 12 hours.

The reaction mixtures were subjected to reversed phase HPLC, and eluted with 0.1 M triethylamine acetate (pH7.0) for 1 hour with a linear gradient of 0 to 60 % acetonitrile at a flow rate of 3.0 mL/min., thereby purifying anthraquinone-modified oligodeoxynucleotide.

The obtained anthraquinone-modifdied oligodeoxynucleotides were 5' -d (T^{AQ}UCACTTCAGTG) - (CH₂)₆-S-S- (CH₂) ₆-T-3' (nucleic acid part is represented by sequence number 1) and 5' -d (T^{AQ}UACACTGAAGTG) - (CH₂) ₆-S-S- (CH₂) ₆-T-3' (nucleic acid part is represented by sequence number 2), respectively.

### EXAMPLE 3

### Preparation of Thiolated Oligodeoxynucleotide

300 µL of a 50 mM sodium phosphate (pH 8.0) solution (0.1 M) of dithiothreitol was added to a 50 mM solium phosphate buffer (pH 8.0) solution (25 µM, total amount 100 µL) of a 3'-disulfide-modified oligodeoxynucleotide, followed by incubation at room temperature for 1 hour. The reaction mixtures were subjected to reversed phase HPLC, and eluted with 0.1 M triethylamine acetate (pH7.0) for 1 hour with a linear gradient of 0 to 60 % acetonitrile at a flow rate of 3.0 mL/min., thereby purifying thiolated anthraquinone-modified oligodeoxynucleotides.

The obtained thiolated anthraquinone-modified oligodeoxynucleotides were 5' -d (T^{AQ}UCACTTCAGTG) - (CH₂) ₆-SH-3' (probe 1)(nucleic acid part is represented by sequence number 1) and 5'-d (T^{AQ}UAUACTGAAGTG) - (CH₂) ₆-SH-3' (probe 2) (nucleic acid part is represented by sequence number 2), respectively.

### < Mass Spectrometry >

The six oligodeoxynucleotide derivatives synthesized in EXAMPLES 2 and 3 were analyzed with MALDI-TOF/MS as below to confirm their structures.

A small amount of solution containing each of the purified oligodeoxynucleotide derivatives was completely digested at 37 °C for 3 hours, using calf intestinal alkaline phosphatase (50 U/ml), snake venom phosphodiesterase (0.15 U/ml), and P1 nuclease (50 U/ml). The digested DNA solutions were each subjected to HPLC on Cosmosil 5C-18AR or on CHEMCOBOND 5-ODS-H columns (4.6 x 150 mm), and eluted with 0.1 M triethylamine acetate (pH7.0) for 20 minutes with a linear gradient of 0 to 20 % acetonitrile at a flow rate of 1.0 mL/min.

The concentration of each oligodeoxynucleotide derivative was determined based on comparison with peak areas of 0.1 mM standard solutions containing dA, dC, dG and dT.

Each oligodeoxynucleotide derivative was analyzed with MALDI-KTOF/MS, obtaining the following results.
(a) Disulfide-Containing Amino-Modified Oligodeoxynucleotide
   5'-d(T^{amino}UCACTTCAGTG)-(CH₂) ₆-S-S-(CH₂) ₆-T-3' (nucleic acid part has a sequence represented by sequence number 1); m/z calculative [M -H]⁻4342.09 was detected at 4342.75.
   5' -d (T^{amino}UACACTGAAGTG) - (CH₂) ₆-S-S- (CH₂) ₆-T-3' (nucleic acid part has a sequence represented by sequence number 2); m/z calculative [M -H]⁻4713.35 was detected at 4715.52.
(b) Anthraquinone-modidfied Oligodeoxynucleotide
   5' -d (T^{AQ}UCACTTCAGTG) - (CH₂) ₆-S-S- (CH₂) ₆-T-3' (nucleic acid part has a sequence represented by sequence number 1); m/z calculative [M-H]⁻4575.30 was detected at 4578.57.
   5'-d (T^{AQ}UACACTGAAGTG) - (CH₂)₆-S-S- (CH₂) ₆-T-3' (nucleic acid part has a sequence represented by sequence number 2); m/z calculative [M -H]⁻4946.56 was detected at 4949.27.
(c) Thiolated Oligodeoxynucleotide
   5'-d(T^{AQ}UCACTTCAGTG)-(CH₂)₆-SH-3' (nucleic acid part has a sequence represented by sequence number 1); m/z calculative [M -H]⁻4138.88 was detected at 4140.67.
   5' -d (T^{AQ}UACACTGAAGTG) - (CH₂) ₆-SH-3' (nucleic acid part has a sequence represented by sequence number 2); m/z calculative [M - H]⁻4507.83 was detected at 4510.48.

### EXAMPLE 4

### Immobilization of Thiolated Oligodeoxynucleotide on Gold Electrode

A gold electrode having an area of 2 mm² was used. Before immobilization of an oligodeoxynucleotide derivative, the electrode was immersed in boiled 2 M potassium hydroxide for 3 hours, and washed with deionized water. Subsequently, the electrode was immersed in concentrated nitric acid for 1 hour, and washed with deionized water.

For chemisorption to the electrode, 1 µL of solutions (10 µM) containing each of two thiolated anthraquinone-modified oligodeoxynucleotides obtained in EXAMPLE 3 (probes 1 and 2) were placed on the electrode, and the end of the electrode was masked with a rubber cap so that the solutions did not evaporate. These electrodes were allowed to stand at room temperature for 2 hours.

Then, to cover the gold surface, 1 µL of a 10 mM Tris-EDTA buffer (pH 8.0) solution (1 mM) containing 6-mercaptohexanol was placed on the gold electrode, and the end of the electrode was masked with a rubber cap so that the solution did not evaporate. These electrodes were allowed to stand at room temperature for 1 hour, and washed with a small amount of deionized water.

### EXAMPLE 5

### Hybridization of Target DNA

To hybridize target DNA with a probe immobilized on the gold electrode, a 1 µL solution containing 10 µM of the target DNA was placed on the gold electrode, and the end of the electrode was masked with a rubber cap so that the solution did not evaporate. The electrode was allowed to stand at room temperature for 30 minutes.

Target DNAs used for probe 1 were sample 1 (3'-AAGTGAAGTCAC-5')(sequence number 3), and sample 2 (3'-AAGTGAAATCAC-5')(sequence number 4). The target DNA represented by sequence number 3 has the base G which corresponds to the base C, base number 8 in sequence number 1 of probe 1, and thus these bases match with each other. The target DNA represented by sequence number 4 has the base A which corresponds to the base C, base number 8 in sequence number 1 of probe 1, and thus these bases mismatch with each other. Sequence numbers 3 and 4 represent partial sequences of aldehyde dehydrogenase (ALDH2), which works in the alcohol metabolic systems. Sequence number 3 has the base G at base number 8; however when this base is replaced with A as in sequence number 4 (G1510A), it is known that such a sequence does not metabolize alcohols well.

Further, target nucleic acids used for probe 1 were sample 3 having the entire length of 91 bases including the base sequence of sample 1 (3'-GGGAGTGGCCGGGAGTTGGGCGAGTACGGGCTGCAGGCATACACTGAAGTGAAAACTGTCACAGT CAAAGTGCCTCAGAAGAACTCATAAG-5') (sequence number 5), and sample 4 having the entire length of 91 bases including the base sequence of sample 2 (3'-GGGAGTGGCCGGGAGTTGGGCGAGTACGGGCTGCAGGCATACACTAAAGTGAAAACTGTCACAGT CAAAGTGCCTCAGAAGAACTCATAAG-5') (sequence number 6). The target DNA represented by sequence number 5 has the base G which corresponds to the base C, base number 46 in sequence number 1 of probe 1, and thus these bases match with each other. The target DNA represented by sequence number 6 has the base A which corresponds to the base C, base number 46 in sequence number 1 of probe 1, and thus these bases mismatch with each other.

Target DNAs for probe 2 were sample 5 (3'-TATGTGACTTCAC-5') (sequence number 7), and sample 6 (3'-TATGTGATTTCAC-5') (sequence number 8). The target DNA represented by sequence number 7 has the base C which corresponds to the base G, base number 8 in sequence number 2 of probe 2, and thus these bases match with each other. The target DNA represented by sequence number 8 has the base T which corresponds to the base G, base number 8 in sequence number 2 of probe 2, and thus these bases mismatch with each other.

The target DNA solution used was an aqueous solution containing 10 mM sodium cacodylate (pH 7.0).

### EXAMPLE 6

### Photo-Electrochemical Measurement

Photo-Electrochemical measurement was performed using a Pyrex® cell equipped with a single compartment. Monochrome excitation light was irradiated through a band pass filter (φ25 mm, Asahi Bunko) having a wavelength of 365 ± 5 nm, using a 200 W UV lamp (Sumida YLT-MX200). Photocurrent was measured at 25 °C, by a three-electrode cell (ALS, Model 660A) consisting of a modified Au working electrode (electrode area 2mm²), a platinum counter electrode, and an SCE reference electrode. Light intensity was calibrated using a UV meter (Ushio, UIT-150). The potocurrent measurement was carried out in a 10 mM sodium cacodylate (pH 7.0) solution using an excitation wavelength of λ = 365 ± 5 nm at a power density of 13 . 0 ± 0.3 mW • cm⁻², in an applied potential to the SCE electrode of + 0.5 V.

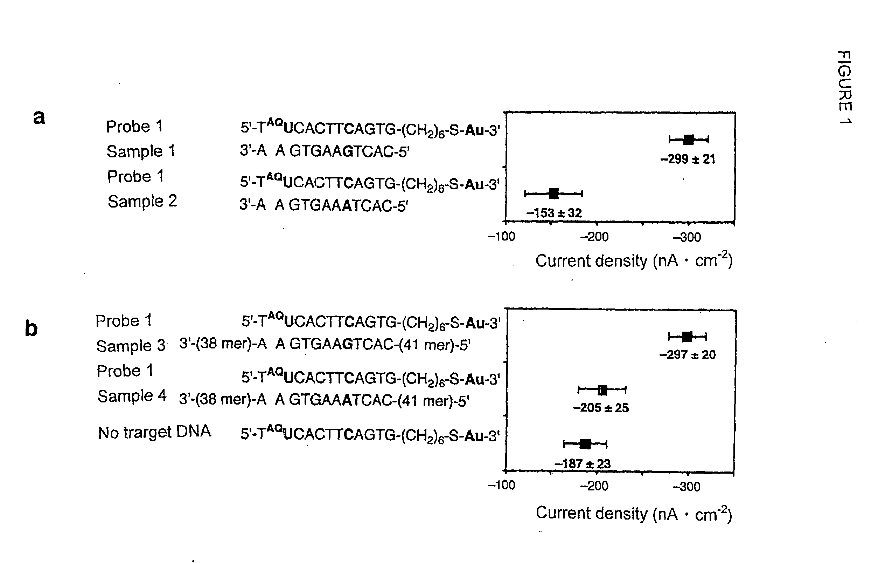

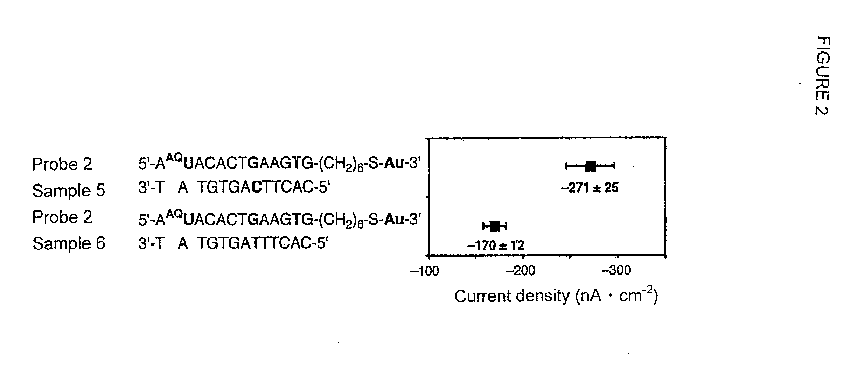

Figures 1a and 1b show the results of probe 1, and FIG. 2 shows the results of probe 2. Figs. 1a and Fig. 2 show the results of 20 experiments, and Fig. 1b shows the results of 10 experiments. Probes 1 and 2 had a current density of -187 ± 23 nA • cm⁻², respectively, before corresponding to the target DNAs.

As shown in Fig. la, probe 1 had a current density of - 299 ± 21 nA. cm⁻² when its base C paired with G of the target DNA (sample 1) consisting of 12 bases. This value was about 1.6 times the current density before the probe corresponded to the target DNA; however, probe 1 had a current density of -153 ± 32 nA • cm⁻² when its base C corresponded to A of the target DNA (sample 2) consisting of 12 bases, and this value was about equal to the current density before the probe corresponded to the target DNA.

As shown in Fig. 1b, probe 1 had a current density of - 297 ± 20 nA cm⁻² when its base C paired with G of the target DNA (sample 3) consisting of 91 bases. This value was about 1.6 times the current density before the probe corresponded to the target DNA; however, probe 1 had a current density of -205 ± 25 nA • cm⁻² when its base C corresponded to A of the target DNA (sample 4) consisting of 91 bases, and this value was about equal to the current density before the probe corresponded to the target DNA. These results reveal that nucleotide(s) can accurately be identified despite target DNA length.

As Fig. 2 shows, probe 2 had a current density of -271 ± 25 nA cm⁻² when its base G paired with C of the target DNA (sample 5). This value was about 1.4 times the current density before the probe corresponded to the target DNA; however, probe 2 had a current density of -170 ± 12 nA cm⁻² when its base G corresponded to T of the target DNA (sample 6), and this value was about equal to the current density before the probe corresponded to the target DNA.

### EXAMPLE 7

### Assay of Oligodeoxynucleotide Immobilized on Gold Electrode

Probes 1 and 2 immobilized on the electrode surface were determined by chronocoulometric assay in the presence of ruthenium (III) hexamine, in accordance with the method by Tarlov et al. ( Steel, A, B.: Herne, T. M.: Tarlov, M. J.: Anal. Chem. 1998, 70, 4670-4677). The electrode having the area of 2 mm² had 6.0 x 10¹²/cm² (9.9 ± 0.8 pmol/cm²) of probes 1 and 2, respectively.

### EXAMPLE 8

### Synthesis of MDA-Containing Nucleoside Derivative

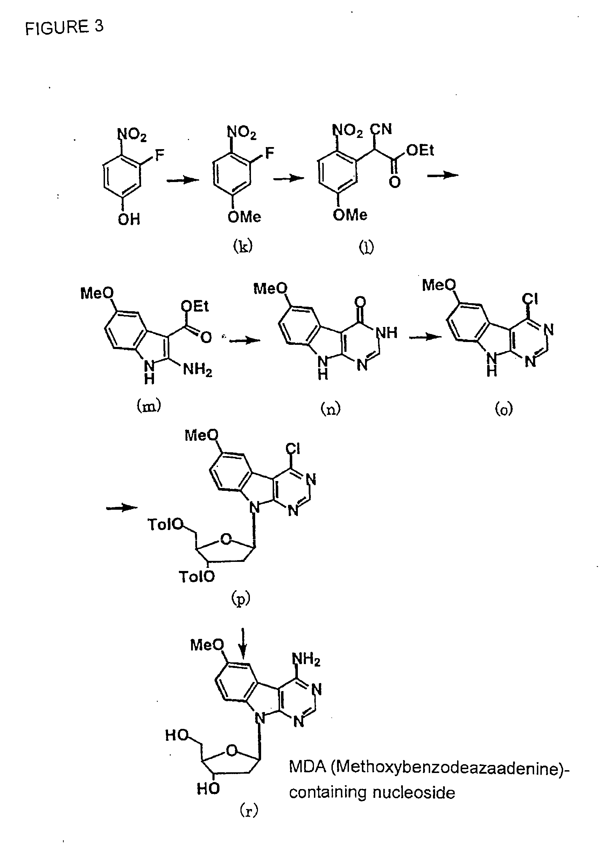

The synthesis of the above derivative is described with reference to Fig. 3. A mixture of 3-fluoro-4-nitrophenol (6.0 g, 38.2 mmol), indomethane (25 ml), and potassium carbonate (10.0 g, 72.0 mmol) in 2-butanone (50 ml) was heated at 40 °C for 4 hours. The reaction mixture was evaporated, and the resultant was added to chloroform (50 ml), thereby obtaining 6.81 g of 3-fluoro-4-nitroanisol (compound (k) in FIG. 3) in the form of a white solid.

Potassium tert-butoxide (10.9 g, 101.9 mmol) was added under nitrogen gas to an ice-cold solution of ethylcyanoacetate (9.8 ml, 93.4 mmol) in anhydrous tetrahydrofuran (THF) (153 ml). The obtained white suspension was stirred for 15 minutes, and compound (k) (8.0 g, 46.7 mmol) was added thereto. The suspension was heated under reflux for 1.5 hours. The solution was added to water, and the aqueous mixture was extracted three times with ether, followed by drying and concentration of its organic compound phase to obtain an oil. Subsequently, the oil was dissolved in chloroform, and eluted with chloroform by column chromatography for purification. The purified fractions were concentrated together, thereby obtaining 14.5 g of cyano (2-nitrophenyl)ethyl acetate ester (compound (1) in Fig.3) in the form of a light yellow oil.

Zn powder (12.1 mg, 185 mmol) was added to a glacial acetic acid (185 ml) solution containing compound (1) (13.2 g, 46.3 mmol). The mixture was sonicated, heated at 55 °C for 45 minutes, and a further 4 g of the Zn powder was added thereto. After sonication, the mixture was further heated for 105 minutes, and the obtained brown mixture was filtered. The filtrate was concentrated to obtain a residue. The residue was purified by column chromatography, and continuously eluted with a hexane solution of 0 %, 5 %, and 10 % ethyl acetate, thereby obtaining 2.07 g of 2-amino-5-methoxy-1H-indole-3-carboxylic acid ethyl ester (compound (m) in Fig.3) (yield of 52 %).

A suspension comprising compound (m) (100 mg, 0,42 mmol), sodium methoxide (50 mg, 0.93 mmol), and formamide (20 ml) was heated at 220 °C for 1.5 hours. The solution was cooled to 25 °C, and added to water. Brown solids were collected and dried, thereby obtaining 1,9-dihydro-6-methoxy-4H-pyrimide[4,5-b]indole-4-one (compound (n) in Fig.3, (47 mg, yield of 52 %).

A mixture comprising compound (n) (500 mg, 2.3 mmol), phosphorylchloride (25 ml), and 1,4-dioxane (25 ml) was refluxed for 6 hours. The mixture was concentrated under reduced pressure, and the residue was added to ethanol. After stirring for 5 minutes, the brown suspension was filtered, and the filtrate was added to water, thereby recovering 4-chloro-6-methoxy-1H-pyrimide [4,5-b]indole (compound (o) in FIG.3) (620 mg, yield of 96 %) in the form of a white solid.

Compound (o) (100 mg, 0.43 mmol) was suspended in anhydrous acetonitrile (10 ml) at room temperature. Sodium hydride (60% in oil, 19 mg, 0.47 mmol) was added to this suspension, and the mixture was stirred under reflux for 10 minutes. Ribose (184 mg, 0.47 mmol) was added thereto, and the mixture was stirred at room temperature for 1 hour. The reaction mixture was concentrated, subjected to silica gel column chromatography, and then eluted with 20 % ethyl acetate in hexane to purify. The obtained product was 210 mg (yield of 82 %) of 4-chloro-6-methoxy-7-(2-deoxy-3,5-di-O-p-toluoyl-β-D-erythropentofuranosyl)-7H-pyrimide[4,5-b]indole (compound (p) in Fig.3) .

Compound (p) (500 mg, 0.85 mmol) in 20 ml methanol/ammonia (saturated at -76 °C) was stirred at 150 °C for 10 hours in a hermetically sealed container. The turbid solution was concentrated, subjected to silica gel column chromatography, and eluted with 10 % methanol in chloroform, thereby obtaining 4-amino-6-methoxy-9-(2'-deoxy-β-D-erythropentofuranosyl)-7H-pyrimide[4,5-b]indole (compound (r) in Fig.3) (420 mg, 75 %).

The thus obtained compound is a deoxyribonucleoside derivative containing methoxybenzodeazaadenine (MDA) as a base.

### EXAMPLE 9

### Synethesis of MDA-Containing Oligonucleotide Derivative

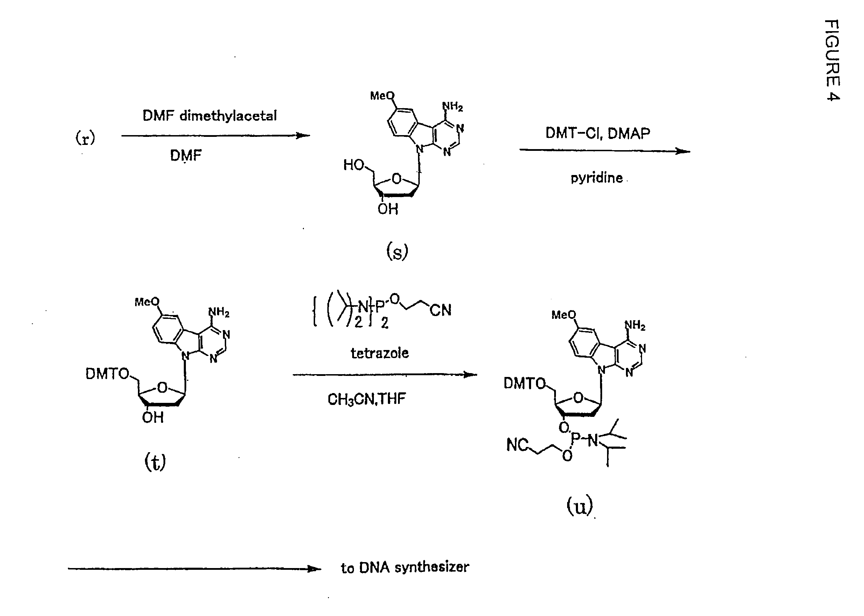

An example of synthesis of an MDA-containing oligonucleotide derivative from the MDA-containing nucleoside derivative obtained in EXAMPLE 8 is described with reference to Fig. 4.

A dimethylformamide (10 ml) solution of compound (r) (90 mg, 0.27 mmol) shown in Fig. 3 containing N,N-dimethylformamide dimethylacetal (10 ml, 28 mmol) was stirred at 60 °C for 3 hours. This solution was concentrated under reduced pressure. The residue was purified using silica gel coloumn chromatography (chloroform:methanol = 10:1) to obtain compound (s) in Fig. 4 in the form of a white solid (120 mg, 0.1 mmol, 88 %).

A pyridine (10 ml) solution of compound (s) (120 mg, 0.1 mmol) containing dimethoxytrithylchloride (DMT-Cl) (137 mg, 0.41 mmol) was stirred at room temperature for 2 hours. The solution was concentrated under reduced pressure. The residue was purified usinsg silica gel chromatography (chloroform:methanol:triethylamine - 90:3:5), thereby obtaining compound (t) in Fig. 4 (161 mg, 75 %) in the form of a white solid.

A 0.3 ml acetonitrile solution containing compound (t) (50 mg, 72.7 mmol), N,N,N',N'-tetraisopropyl-2-cyanoethylphosphoroamidite (25 µl, 79. 9 µmol), and tetrazol (7 mg, 0.1 mmol) was stirred at room temperature for 2 hours. The mixture was filtered, thereby obtaining compound (u) shown in Fig. 4. The thus obtained compound (u) was used for DNA synthesis without further purification.

Compound (u) was used for synthesizing an oligodeoxyribonucleotide derivative 5'-A^{AQ}UACACTnAAGTG-3' (sequence number 7)(n is an MDA-containing derivative; equivalent to compound (u)) by phosphoroamidite method using an Applied Biosystem 392 DNA/RNA synthesizer. The obtained MDA-containing oligodeoxyribonucleotide derivative was purified using reversed phase HPLC on 5-ODS-H column (10 x 150 mm). The elution was performed with 0.1 M triethylamine acetate (TEAA), pH7.0, for 20 minutes with a linear gradient of .5 to 25 % acetonitrile at a flow rate of 3.0 mL/min.

### EXAMPLE 10

### Photo-Electrochemical Measurement

Probe 3 containing an MDA-containing oligonucleotide was immobilized on a gold electrode in the same manner as in EXAMPLES 2 to 4, using the oligodeoxyribonucleotide derivative obtained in EXAMPLE 9. The base sequence of the nucleic acid part of probe 3 was 5'-A^{AQ}UACACT^{MD} AAAGTG-3' (sequence number 9).

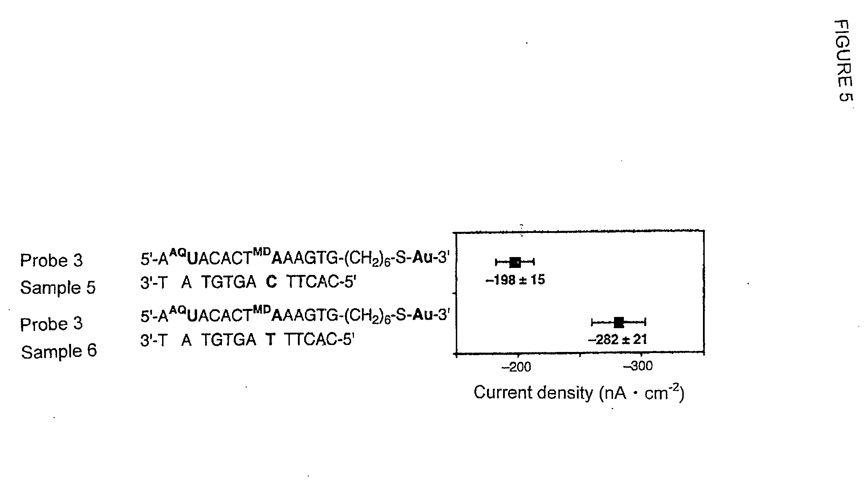

Probe 3 was hybridized with sample 5 (sequence number 7) and sample 6 (sequence number 8), respectively, in the same manner as in EXAMPLE 5. The target DNA represented by sequence number 7 has the base C which corresponds to MDA, base number 8 of probe 3, and thus these bases match with each other. The target DNA represented by sequence number 8 has the base T which corresponds to MDA, base number 8 of probe 3, and thus these bases mismatch with each other. Further, photo-Electrochemical measurement was performed in the same manner as in EXAMPLE 6. Fig. 5 shows results thereof.

Probe 3 had a current density of -187 ± 23 nA • cm⁻² before corresponding to the target DNA. Probe 3 had a current density of -282 ± 21 nA • cm⁻² when its MDA-containing nucleotide was paired with the base T of the the target DNA (sample 6), and this value was about 1.5 times the current density before the probe corresponded to the target DNA; however, the current density was -198 ± 15 nA • cm⁻² when the MDA-containing nucleotide corresponded to the base C of the target DNA (sample 5), and this value was about equal to the current density before the probe corresponded to the target DNA.

### EXAMPLE 11

Probe 4 having a nucleic acid part represented by the base sequence 5'-I^{AQ}UAGACAT^{MD} AAC-3' (sequence number 10) was immobilized on a gold electrode in the same manner as in EXAMPLES 2 to 4. The base at base number 9 of probe 4 needs to be complementary to the base T of the target DNA; however the base A at base number 9 is replaced with an MDA-containing nucleotide so that probe 4 does not have four or more bases A or T continuously. Therefore, the MDA-containing nucleotide at base number 9 is not for detecting polymorphisms.

Probe 4 was hybridized with sample 7 (3'-CATCTGTATTG-5'; sequence number 11) and sample 8 (3'-CATCTATATTG-5'; sequence number 12), respectively, in the same manner as in EXAMPLE 5. Samples 7 and 8 are partial sequences of human thiopurine s-methyltransferase (TPMT) gene. TPMT is relevant to the metabolism of azathiopurine and 6-mercaptopurine, which is drugs as drugs to treat blood malignant tumors. TPMT gene polymorphism is frequently present, and the most common mutant allele among the Japanese people is TPMT*3C. Mutant TPMT*3C has replaced the base A at base number 6 with G, and it is known that this substitution makes it harder to metabolize the above drugs.

The target DNA represented by sequence number 11 has a base G which corresponds to the base C at base number 6 of probe 4, and thus these bases match with each other; however, the target DNA represented by sequence number 12 has a base A which corresponds to the base C at base number 6 of probe 4, and thus these bases mismatch with each other.

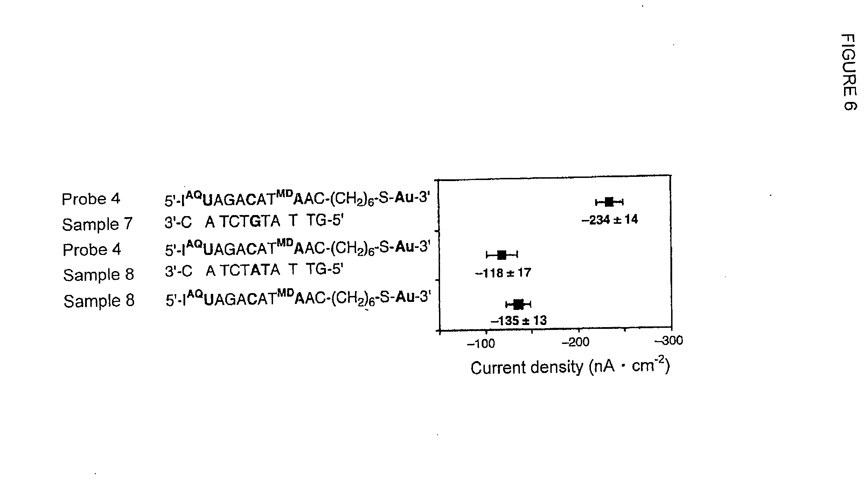

Further, photo-electrochemical measurements were performed in the same manner as in EXAMPLE 6. Fig. 6 shows results obtained from 10 experiments. Probe 4 had a current density of -135 ± 13 nA. cm⁻² before corresponding to the target DNAs . The current density was -234 ± 14 nA. cm⁻² when the base C of probe 4 was paired with the base G of the target DNA (sample 7), and this value was about 1.7 times the current density before the probe corresponded to the target DNA; however, the current density was -118 ± 17 nA • cm⁻² when the base C of probe 4 corresponded to the base A of the target DNA (sample 8), and this value was about equal to the current density before the probe corresponded to the target DNA.

### EXAMPLE 12

From 25 consenting panelists, a region consisting of 91 bases containing the G1459A site of ALDH2 gene in their chromosomal DNA was amplified by PCR using a forward primer (5'-GGGAGTGGCCGGGAGTT-3')(sequence number 13) and a reverse primer (5'-CTTATGAGTTCTTCTGA-3')(sequence number 14), thereby obtaining target DNAs.

G probes (base sequence of nucleic acid part: 5'-A^{AQ}UACACTGAAGTG (sequence number 15) for detecting a base C or G, and MDA probes (base sequence of nucleic acid part: 5'-A^{AQ}UACACT^{MD} AAAGTG (sequence number 16)) for detecting a base T or A were immobilized on gold electrodes in the same manner as in EXAMPLES 3 and 4. These probes were hybridized with target DNAs, respectively, in the same manner as in EXAMPLE 5, then subjected to photo-electrochemical measurements in the same manner as in

### EXAMPLE 6.

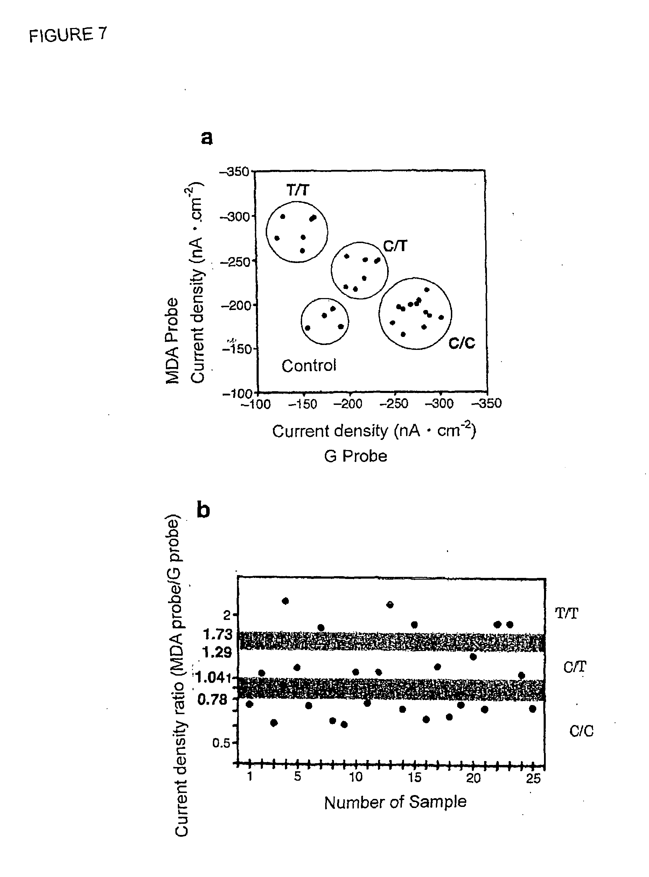

Figure. 7a shows results of the plotted current densities detected when using MDA probes against those detected when using G probes. The control in Fig. 7a shows the current densities detected before each probe was hybridized with the corresponding target DNA.

Figure. 7b shows the ratio values of the current densities detected when using MDA probes against current densities detected when using G probes. In Fig. 7b, the above ratio values between 1.04 and 1.29 mean a C/T heterozygous group, those of 0.78 or below mean a C/C homozygous group, and those of 1.73 or higher mean a T/T homozygous group.

As Figs. 7a and 7b show, measurement of the current densities using both probes clearly separated 25 panelists into T/T homozygous, C/C homozygous, and C/T heterozygous groups. Further, since the measurement results using both probes were correlative, nucleotide identification methods using these probes are revealed to be reliable.

## Claims

1. A probe unit for identifying a target nucleotide region in a target nucleic acid, the probe unit being provided with an electrode, a probe bound to the electrode and recognizes the target nucleic acid, and a hole transfer-inducing agent bound to the probe, wherein a nucleotide region corresponding to the target nucleotide region is located between the hole-transfer-inducing-agent-binding site and the electrode-binding end of the probe.

2. A probe unit for identifying a target nucleotide in a target nucleic acid, the probe unit being of Claim 1, wherein a nucleotide corresponding to the target nucleotide is located between the hole-transfer-inducing-agent-binding site and the electrode-binding end of the probe.

3. A probe unit of Claim 1, wherein the probe recognizes the target nucleic acid by the hybridization therewith.

4. A probe unit of Claim 1, wherein the probe consists of 6 to 30 nucleotides or derivatives thereof.

5. A probe unit of Claim 1, wherein the hole-transfer-inducing agent is a photosensitizer.

6. A probe unit of Claim 5, wherein the photosensitizer is capable of causing a photoexcited hole transfer.

7. A probe unit of Claim 5, wherein the photosensitizer is at least one selected from the group consisting of quinone photosensitizers, flavin photosensitizers, and benzophenone photosensitizers.

8. A probe unit of Claim 1, wherein the probe is bound to the electrode via a spacer.

9. A probe unit of Claim 8, wherein the spacer is a substance selected from the group consisting of organic low molecular weight compounds, nucleic acids, and polypeptides.

10. A probe unit of Claim 8, wherein the spacer has a length of 1 to 3 nm.

11. A probe unit of Claim 1, wherein the electrode is provided with a plurality of electrode spacers on its spacer-binding face.

12. A probe unit of Claim 2, wherein the probe is DNA, and a base of the nucleotide corresponding to the target nucleotide in the probe is guanine or cytosine.

13. A probe unit of Claim 2, wherein the probe is an oligonucleotide derivative including a nucleotide derivative represented by formula (1) below at a site corresponding to the target nucleotide of the target nucleic acid 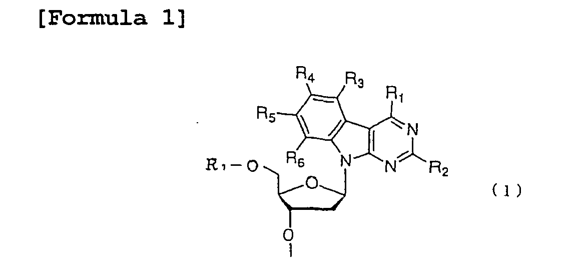 wherein R₁, R₂, R₃, R₄, R₅ and R₆ each independently represent hydrogen, an amino group, a lower mono-alkylamino group, a lower di-alkylamino group, a hydroxyl group, a lower alkoxy group, a halogen atom, a cyano group, a mercapto group, a lower alkylthio group or an aryl group.

14. A probe unit of Claim 1, wherein a number of continuous nucleotides selected from the group consisting of A and T in the probe is 3 or less.

15. A probe unit of Claim 1, wherein the nucleotides at the opposite side to the electrode-binding end from the nucleotide to which the hole-transfer-inducing agent is bound in the probe are nucleotides other than G and C.

16. A probe unit of Claim 1, wherein the hole-transfer-inducing agent is bound to a nucleotide in the probe.

17. An apparatus for identifying a nucleotide region being provided with a substrate, at least one probe unit of any of Claims 1 to 16, and a means for detecting an electrochemical signal from the electrode of the probe unit, wherein the electrode of the probe unit is positioned on the substrate so as to detect the electrochemical signal.

18. A method for identifying a target nucleotide region, the method comprising:
a first step in which a target nucleic acid and a probe bound to an electrode are hybridized;
a second step in which a hole-transfer-inducing agent is directly or indirectly bound to the probe or target nucleic acid before or after the first step, during which the hole-transfer-inducing agent is bound to the probe so that a nucleotide region corresponding to the target nucleotide region is located between the hole-transfer-inducing-agent-binding site and electrode-binding site of the probe, or the hole-transfer-inducing agent is bound to the target nucleic acid so that the target nucleotide region is located between a site corresponding to the electrode-binding site of the probe and the hole-transfer-inducing-agent-binding site in the target nucleic acid; and
a third step in which a hole transfer from the hole-transfer-inducing-agent-binding site to the electrode in the probe is caused by an energy supply to the above hybridized product, an electrochemical signal detected from the electrode is detected, the signal is compared with an electrochemical signal detected from the electrode caused by an energy supply to the probe before the hybridization, and whether or not the target nucleotide region in the target nucleic acid is completely complementary to the corresponding region in the probe is identified based on this comparison.

19. A method of Claim 18, wherein the hole-transfer-inducing agent is bound to the probe.

20. A method of Claim 18, wherein the second step is performed before the first step.

21. A method of Claim 18, wherein the target nucleotide region consists of a single target nucleotide.

22. A method of Claim 18, wherein the probe consists of 6 to 30 nucleotides or derivatives thereof.

23. A method of Claim 18, wherein the hole-transfer-inducing agent is a photosensitizer.

24. A method of Claim 23, wherein the photosensitizer is capable of causing a photoexcited hole transfer.

25. A method of Claim 23, wherein the photosensitizer is at least one selected from the group consisting of quinone photosensitizers, flavin photosensitizers, and benzophenone photosensitizers.

26. A method of Claim 18, wherein the probe is bound to the electrode via a spacer.

27. A method of Claim 26, wherein the spacer is a substance selected from the group consisting of organic low molecular weight compounds, nucleic acids, and polypeptides.

28. A method of Claim 26, wherein the spacer has a length of 1 to 3 nm.

29. A method of Claim 18, wherein the electrode is provided with a plurality of electrode spacers on its spacer-binding face.

30. A method of Claim 21, wherein the probe is DNA, and a base of the nucleotide corresponding to the target nucleotide in the probe is guanine or cytosine, the method identifying whether the base of the target nucleotide in the target nucleic acid is cytosine or guanine in the third step.

31. A method of Claim 21, wherein the probe is an oligonucleotide derivative containing a nucleotide derivative represented by formula (1) below at a site corresponding to the target nucleotide in the target nucleic acid, the method identifying whether or not the base of the target nucleotide in the target nucleic acid is thymine in the third step 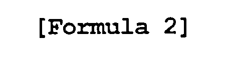 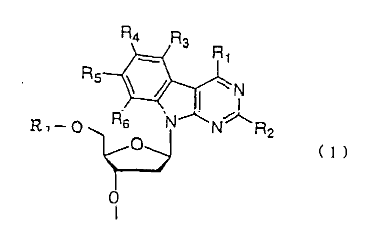 wherein R₁, R₂, R₃, R₄, R₅ and R₆ each independently represent hydrogen, an amino group, a lower mono-alkylamino group, a lower di-alkylamino group, a hydroxyl group, a lower alkoxy group, a halogen, a cyano group, a mercapto group, a lower alkylthio group or an aryl group.

32. A method of Claim 18, wherein a number of continuous nucleotides selected from the group consisting of A and T in the probe is 3 or less.

33. A method of Claim 18, wherein the nucleotides at the opposite side to the electrode-binding end from the nucleotide to which the hole-transfer-inducing agent is bound in the probe are nucleotides other than G and C.

34. A method of Claim 18, wherein the hole transfer-inducing agent is bound to the nucleotide in the probe.

35. A method of Claim 18, wherein energy is irradiated at a power density of 1 to 100 mW•cm⁻².

36. A method of Claim 23, wherein light having a wavelength of 300 to 600 nm is irradiated.

37. A method for identifying a target nucleotide region comprising:
a step of hybridizing the probe in the probe unit of Claim 12 and a target nucleic acid; and
a step of detecting an electrochemical signal from the electrode by an energy supply to the probe, comparing the signal with an electrochemical signal detected from the electrode when energy is supplied to a probe with which the target nucleic acid is not hybridized, and identifying whether or not a target nucleotide region in the target nucleic acid is completely complementary to a corresponding region in the probe based on this comparison.

38. A method of Claim 37, wherein the target nucleotide region consists of a single nucleotide, the method identifying, in the identification step, whether or not the base of the target nucleotide in the target nucleic acid is cytosine or guanine by identifying whether or not the target nucleotide in the target nucleic acid is complementary to the corresponding region in the probe.

39. A method of Claim 37, wherein energy is irradiated at 1 to 100 mW • cm⁻².

40. A method of Claim 37, wherein the energy is light, and light having a wavelength of 300 to 600 nm is irradiated.

41. A method for identifying a target nucleotide region comprising:
a step of hybridizing the probe in the probe unit of Claim 13 and a target nucleic acid; and
a step of detecting an electrochemical signal from the electrode by an energy supply to the probe, comparing the signal with an electrochemical signal detected from the electrode when energy is supplied to a probe with which the target nucleic acid is not hybridized, and identifying whether or not a target nucleotide region in the target nucleic acid is completely complementary to a corresponding region in the probe based on this comparison.

42. A method of Claim 41, wherein a target nucleotide region consists of a single nucleotide, the method identifying, in the identification step, whether or not the base of the target nucleotide in the target nucleic acid is thymine by identifying whether or not the target nucleotide in the target nucleic acid is complementary to the corresponding region in the probe.

43. A method of Claim 41, wherein energy is irradiated at 1 to 100 mW ·cm⁻².

44. A method of Claim 41, wherein the energy is light, and light having a wavelength of 300 to 600 nm is irradiated.

45. A method for producing a probe unit for identifying a target nucleotide region in a target nucleic acid, the method comprising the steps of binding a hole-transfer-inducing agent directly or indirectly to a probe that recognizes a target nucleic acid, and of binding an end of the probe to an electrode, the hole-transfer-inducing agent being bound during the hole-transfer-inducing-agent binding step so that a nucleotide region corresponding to the target nucleotide region is located between the hole-transfer-inducing-agent-binding site and electrode-binding end of the probe.


SEQUENCE LISTING

<110>  Kyoto University

<120>  Probe unit, apparatus for identifying a nucleotide region, and method for
identifying a nucleotide region

<130>  P05-80

<150>  JP2004-369133
<151>  2004-12-21

<160>  16

<170>  PatentIn version 3.1

<210>  1
<211>  12
<212>  DNA
<213>  Artificial sequence

<220>
<223>  Oligonucleotide used as a probe

<400>  1
tucacttcag tg                                                          12


<210>  2
<211>  13
<212>  DNA
<213>  Artificial sequence

<220>
<223>  Oligonucleotide used as a probe

<400>  2
tuacactgaa gtg                                                         13


<210>  3
<211>  12
<212>  DNA
<213>  Human

<400>  3
aagtgaagtc ac                                                          12


<210>  4
<211>  12
<212>  DNA
<213>  Human

<400>  4
aagtgaaatc ac                                                          12


<210>  5
<211>  91
<212>  DNA
<213>  Human

<400>  5
gggagtggcc gggagttggg cgagtacggg ctgcaggcat acactgaagt gaaaactgtc      60

acagtcaaag tgcctcagaa gaactcataa g                                     91

<210> 6
<211> 91
<212> DNA
<213> Human

<400> 6
gggagtggcc gggagttggg cgagtacggg ctgcaggcat acactaaagt gaaaactgtc 60

acagtcaaag tgcctcagaa gaactcataa g 91


<210> 7
<211> 13
<212> DNA
<213> Human

<400> 7
tatgtgactt cac 13


<210> 8
<211> 13
<212> DNA
<213> Human

<400> 8
tatgtgattt cac 13


<210> 9
<211> 13
<212> DNA
<213> Artificial sequence

<220>
<223> Oligonucleotide used as a probe

<220>
<221> misc_feature
<222> (8)..(8)
<223> n stands for MDA-containing nucleotide


<400> 9
auacactnaa gtg 13


<210> 10
<211> 11
<212> DNA
<213> Artificial sequence

<220>
<223> Oligonucleotide used as a probe

<220>
<221> misc_feature
<222> (1)..(1)
<223> n stand for inosine-containing nucleotide


<220>
<221> misc_feature
<222> (9)..(9)
<223> n stands for MDA-containing nucleotide

<400>  10
nuagacatna c                                                            11


<210>  11
<211>  11
<212>  DNA
<213>  Human

<400>  11
catctgtatt g                                                            11


<210>  12
<211>  11
<212>  DNA
<213>  Human

<400>  12
catctatatt g                                                            11


<210>  13
<211>  17
<212>  DNA
<213>  Artificial sequence

<220>
<223>  Oligonucleotide used as a probe

<400>  13
gggagtggcc gggagtt                                                      17


<210>  14
<211>  17
<212>  DNA
<213>  Artificial sequence

<220>
<223>  Oligonucleotide used as a probe

<400>  14
cttatgagtt cttctga                                                      17


<210>  15
<211>  13
<212>  DNA
<213>  Artificial sequence

<220>
<223>  Oligonucleotide used as a probe

<400>  15
auacactgaa gtg                                                          13


<210>  16
<211>  13
<212>  DNA
<213>  Artificial sequence

<220>
<223>  Oligonucleotide used as a probe

<220>
<221>  misc_feature

<222>  (8)..(8)
<223>  n stands for MDA-containing nucleotide

<400>  16
auacactnaa gtg                                                          13